(19) Europäisches Patentamt
European Patent Office
Office européen des brevets

(11) Publication number: **0 159 684**
**B1**

(12) **EUROPEAN PATENT SPECIFICATION**

(45) Date of publication of patent specification: **27.09.89**

(51) Int. Cl.$^4$: **C 07 D 313/12,** C 07 C 69/76, A 61 K 31/335

(21) Application number: **85104829.8**

(22) Date of filing: **20.04.85**

(54) **(6,11-Dihydro-11-oxodibenz(b,e)oxepinyl)pentanoic acids and derivatives, a process and intermediates for their preparation and their use as medicaments.**

(30) Priority: **27.04.84 US 604672**

(43) Date of publication of application:
**30.10.85 Bulletin 85/44**

(45) Publication of the grant of the patent:
**27.09.89 Bulletin 89/39**

(84) Designated Contracting States:
**AT BE CH DE FR GB IT LI LU NL SE**

(56) References cited:
**DE-A-2 716 230**
**FR-A-2 319 339**

(73) Proprietor: **HOECHST-ROUSSEL PHARMACEUTICALS INCORPORATED**
**Route 202-206 North**
**Somerville New Jersey 08876 (US)**

(72) Inventor: **Martin, Lawrence L.**
**R.D.3, Concord Road Box 81**
**Lebanon New Jersey 08833 (US)**
Inventor: **Setescak, Linda L.**
**Kimberly Road 102**
**Somerville New Jersey 08876 (US)**

(74) Representative: **Meyer-Dulheuer, Karl-Hermann, Dr. et al**
**HOECHST Aktiengesellschaft Postfach 3540**
**D-6121 Wiesbaden (DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to (6,11-dihydro-11-oxodibenz[b,e]oxepinyl)pentanoic acids and derivatives thereof. More particularly, the present invention relates to (6,11-dihydro-11-oxodibenz[b,e]oxepinyl)pentanoic acids of formula

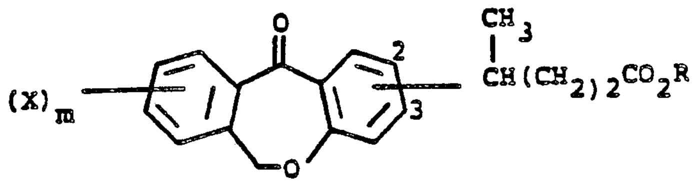

I

wherein the group

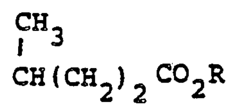

is bound to either the 2- or 3-position of the dibenz[b,e]oxepin ring, and wherein R is hydrogen or alkyl of 1 to 5 carbon atoms; X alkyl of 1 to 5 carbon atoms, alkoxy of 1 to 5 carbon atoms, halogen or trifluoromethyl; and m is 0, 1 or 2, the optical antipode thereof or a pharmaceutically acceptable salt thereof, which are useful as antiarthritic agents, alone or in combination with inert pharmaceutical carriers.

FR—A—2 319 339 discloses compounds of the formula

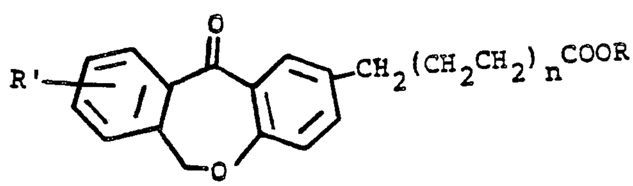

where n is 1, 2 or 3, which exhibit anti-inflammatory and analgesic activities.

Preferred compounds of the present invention are those wherein R and X are hydrogen.

The present invention also relates to alkyl (2-carboalkoxy-benzyloxy)phenyl pentanoates of formula

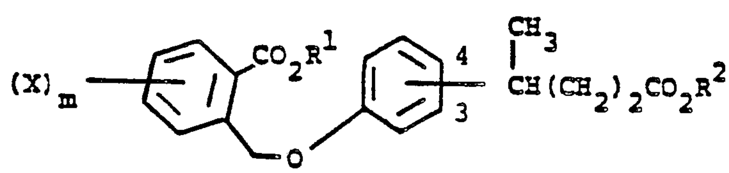

II

wherein the group

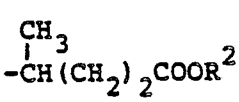

is bound to either the 3- or 4-position wherein $R^1$ is hydrogen or alkyl of 1 to 5 carbon atoms; $R^2$ is hydrogen or alkyl of 1 to 5 carbon atoms with the proviso that $R^1$ and $R^2$ are simultaneously hydrogen or alkyl of 1 to 5 carbon atoms; X is alkyl of 1 to 5 carbon atoms; alkoxy of 1 to 5 carbon atoms, halogen or trifluoromethyl; and m is 0, 1 or 2, or the optical antipode thereof, and alkyl (hydroxyphenyl)pentanoates of the formula 3

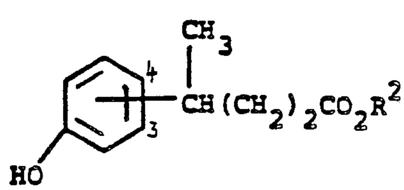

3

wherein the group

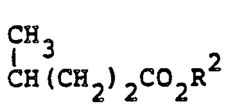

is bound to either the 3- or 4-position and, wherein $R^2$ is alkyl 1 to 5 carbon atoms, or an optical antipode

thereof, useful as intermediates for the preparation of the hereinbeforementioned (6,11-dihydro-11-oxodibenz[b,e]oxepinyl)pentanoic acids and derivatives thereof.

Preferred alkyl [(2-carboalkoxybenzyloxy)phenyl]-pentanoates are those wherein $R^2$ is alkyl of 1 to 5 carbon atoms, and m is 0.

As used through the specification and appended claims, the term "alkyl" refers to a straight or branched chain hydrocarbon radical containing no unsaturation and having 1 to 5 carbon atoms such as methyl, ethyl, 1-propyl, 2-propyl, 1-butyl, 1-pentyl, 2-pentyl, and the like; the term "alkoxy" refers to a monovalent substituent which consists of an alkyl group linked through an ether oxygen and having its free valence bond from the ether oxygen such as methoxy, ethoxy, propoxy, butoxy, 1,1-dimethylethoxy, pentoxy. The term "alkanoic acid" refers to a compound formed by combination of a carboxyl group with a hydrogen atom or alkyl group. Examples of alkanoic acids are formic acid, acetic acid, propanoic acid, 2,2-dimethylacetic acid, pentanoic acid; the term "halogen" refers to a member of the family fluorine, chlorine, bromine or iodine.

The compounds of the present invention which lack an element of symmetry exist as optical antipodes and as the racemic forms thereof. The optical antipodes may be prepared from the corresponding racemic forms by standard optical resolution techniques, involving, for example, the separation of diastereomeric salts of those instant compounds characterized by the presence of an acidic carboxylic acid group and an optically active base, or by the synthesis from optically active precursors.

The present invention comprehends all optical isomers and racemic forms thereof of the compounds disclosed and claimed herein. The formulas of the compounds shown herein are intended to encompass all possible optical isomers of the compounds so depicted.

The novel (6,11-dihydro-11-oxodibenz[b,e]oxepinyl)pentanoic acids *1* of the present invention are synthesized by the process illustrated in Reaction Scheme A.

Condensation of a benzylhaloester *4* with a phenolic ester *3* provides a benzyloxyester *2* which is hydrolyzed to a benzyloxyacid *5* and, in turn, cyclized to a (6,11-dihydro-11-oxodibenz[b,e]oxepinyl)-pentanoic acid *6*. The pentanoic acid *6* may be esterified to the ester *8*.

The condensation is accomplished by contacting a benzylhalo-ester *4* with a phenolic ester *3* in an alkanone in the presence of an alkali metal carbonate. Among alkanones there may be mentioned 2-propanone, 2-butanone, 3-pentanone. Among alkali metal carbonates there may be mentioned lithium, sodium and potassium carbonate. 2-Butanone and potassium carbonate are the preferred alkanone and alkali metal carbonate, respectively. The temperature at which the condensation is conducted is not critical. However, to ensure a reasonable rate of reaction, the condensation is preferably performed at the reflux temperature of reaction medium.

A condensation promoter such as an alkali metal halide, for example, lithium, sodium or potassium bromide, or lithium, sodium or potassium iodide, may be employed. Potassium iodide is the preferred promoter.

The hydrolysis is affected by treating a diester *2* with an alkali metal hydroxide such as lithium hydroxide, sodium hydroxide, potassium hydroxide and the like in an aqueous alkanol such as methanol, ethanol, 1- and 2-propanol, 2,2-dimethylethanol, pentanol, and the like. Potassium hydroxide and aqueous ethanol is the preferred reaction medium. The hydrolysis may be accomplished at ordinary temperatures. To facilitate the reaction, however, elevated temperatures falling within the range of ambient temperature to the reflux temperature of the reaction medium may be employed. The reflux temperature of the reaction medium is preferred.

The cylization is preferably conducted by contacting a diacid *5* with trifluoroacetic anhydride in a halocarbon solvent, such as dichloromethane, trichloromethane, tetrachloromethane, 1,1-dichloromethane, 1,2-dichloromethane, and the like. The cyclization temperature is not narrowly critical. The reaction is accomplished at ambient temperature (25°C) to the reflux temperature of the reaction medium. The reflux temperature of the reaction medium is preferred.

The cyclization proceeds efficiently when two or more molar-equivalents or trifluoroacetic anhydride (relative to diacid 5) are employed. It is preferable to conduct the cyclization employing about two to about six molar-equivâlents of trifluoroacetic anhydride, and most preferable to employ about three molar equivalents of the cyclizing agent.

The cyclization of a diacid *5* to an oxepinonepentanoic acid *6* may be affected by means of phosphorus-containing agents such as phosphorus pentoxide, phosphorus pentoxide-alkanol, polyphosphoric acid-alkanoic acid and the like. For example, treatment of a diacid *5* with phosphorus pentoxide or phosphorus pentoxide-ethanol in the presence or absence of a solvent, such as sulfolane, at ambient to moderate temperatures within the range of about 25° to 120°C, (a cyclization temperature of about 90°C being preferred), and a treatment of diacid *5* with polyphosporic acid or polyphosphoric acid-acetic acid at ambient to moderate temperatures within the range of about 25° to 100°C, (a reaction temperature of about 80°C being preferred), affords oxepinone *6*.

The cyclization of diacid *5* to oxepinonepentanoic acid *6 via* diacid halide *7* may also be achieved by means of Friedel-Crafts reagents. For example, treatment of diacid cloride *7*, which may be prepared from diacid *5* by contacting it with a halogenating agent such as phosphorus trichloride or thioniyl chloride, with catalytic or excess amounts (e.g., about 0.001 to about 10 molar-equivalents of Friedel-Crafts reagent to diacid cloride *7*) of aluminum chloride, stannic chloride, ferric chloride, and the like in a halocarbon solvent

(e.g., dichlormethane, 1,2-dichloromethane, 1,1,2-trichloroethylene, and the like) at reduced to moderate temperatures (e.g., about 0° to about 120°C) yields oxepinonepentanoic acid *6*, after hydrolysis.

A (6,11-dihydro-11-oxodibenz[b,e]oxepinyl)pentanoic acid *6* may be transformed to the corresponding ester *8*, i.e. an alkyl (6,11-dihydro-11-oxodibenz[b,e]oxepinyl)pentanoate of formula *8*, by esterification procedures. For example, a pentanoic acid *6* may be treated with an alkanol in the presence of a mineral acid such as sulfuric acid, hydrochloric acid, hydrobromic acid, and the like, or a strong organic acid such as methanesulfonic acid, 4-methylphenylsulfonic acid, and the like to afford ester *8*.

Benzylhaloester *4* is prepared by procedures described and referenced in D. E. Aultz, et al., J. Med. Chem., *20*, 66 (1977).

Phenolic ester *3* is prepared by the sequence depicted in Scheme B, involving esterification of nitrophenylpentanoic acid *9* to alkyl nitrophenylpentanoate 10, followed by reduction of alkyl nitrophenylpentanoate 10 to alkyl aminophenylpentanoate *11* and conversion of alkyl aminophenyl pentanoate *11* to alkyl hydroxyphenylpentanoate *3*. Thus, treatment of acid *9* with an alkanol, e.g. ethanol, in the presence of a mineral acid, e.g., sulfuric acid, provides ester *10*, which is reduced to aniline *11* with hydrogen in the presence of a hydrogenation catalyst, e.g., palladium-on-carbon, and, in turn, diazotized with nitrous acid, generated *in situ* from an alkali metal nitrite, e.g., sodium nitrite, and a mineral acid, e.g., sulfuric acid, to a diazonium salt and, without isolation, hydrolized to the phenol *3* by means of aqueous copper sulfate solution.

The (6,11-dihydro-11-oxodibenz[b,e]oxepinyl)pentanoic acids and derivatives thereof of the present invention are useful as antiarthritic agents due to their ability to suppress arthritis-type inflammation in mammals. The antiarthritic utility is demonstrated in the adjuvant-induced polyarthritis syndrome in rats, by a procedure similar to that described by C. M. Perron and F. D. Wood, Arthritis and Rheumatism, *2*, 440 (1959).

Groups of 10 male Charles River-Wistar Lewis rats weighing 150 to 175 g were individually housed and maintained on a regular rat chow diet. Water was given *ad libitum.* The adjuvant was prepared by suspending 75 mg of *Mycobacterium butyricum* (Defco Laboratories, Detroit, Michigan) in 10 ml of white paraffin oil with continuous stirring for 2 hrs at room temperature prior to administration. Test compounds are prepared by suspending the drug in water, adding one drop of Tween 80 per 10 ml of suspension, and homogenizing. The adjuvant suspension (0.1 ml) was injected into the footpad of the left hind paw of the rat. Test compound suspensions were administered orally (10 ml/kg) the day before adjuvant suspension injection and the administration was continued for 21 days. One groups of ten rats was used for the test drug, standard, adjuvant-injected control and non-injected control. Control animal received vehicle (10 mg/kg). Three doses of test drug and one dose of standard preparation were used. Injected and non-injected paw volumes were determined on the day the adjuvant suspension was given, and on subsequent days thereafter (usually days 5, 10, 18, and 21) by the method of C. A. Winter, et al., Proc. Soc. Exp. Biol. Med., *111*, 544 (1962).

The percent inhibition of paw volume (injected and non-injected hind paw) was calculated by the following formula:

$$\%\ \text{Inhibition} = \frac{\begin{matrix}\text{Mean Paw Volume} \\ \text{Change of Injected}\end{matrix} - \begin{matrix}\text{Mean Paw Volume} \\ \text{Change of Drug}\end{matrix} \\ \text{(or non-injected) Control Treated}}{\text{Mean Paw Volume Change of Injected (or Non-Injected Control)}} \times 100$$

$ED_{50}$-values, i.e., the dose at which the drugs affects a 50% inhibition of paw volume, were estimated by method of J. T. Litchfield and F. Wilcoxon, J. Pharm. Exp. Ther., *96*, 99 (1948) and statistically evaluated by means of the student "t" test. $ED_{50}$-values of a compound of the present invention and a standard are presented below:

| Compound | $ED_{50}$ (mg/kg/day) |
|---|---|
| 4-(6,11-dihydro-11-oxodibenz[b,e]-oxepin-2-yl)pentanoic acid | 6.4 (injected)<br>3.4 (non-injected) |
| Phenylbutazone | 12 (injected)<br>6.8 (non-injected) |

Arthritic-like inflammation suppression is achieved when the present (6,11-dihydro-11-oxidibenz[b,e]oxepinyl)pentanoic acids are administered to a subject requiring such treatment as an effective oral, parenteral or intravenous dose of from 0.01 to 100 mg/kg of body weight per day. A particularly effective amount is about 25 mg/kg of body weight per day. It is to be understood, however, that for any particular subject, specific dosage regimens should be adjusted according to the individual need and the professional judgement of the person administering or supervising the administration of the

aforesaid compound. It is to be further understood that the dosages set forth herein are exemplary only and that they do not, to any extent, limit the scope or practice of the invention.

Effective amounts of a compound of the present invention may be administered to a subject by any one of various methods, for example, orally as in capsules or tablets, parenterally in the form of sterile solutions or suspensions, and in some cases intravenously in the form of sterile solutions. The (6,11-dihydro-11-oxodibenz[b,e]oxepinyl)pentanoic acids of the present invention form addition salts with pharmaceutically acceptable non-toxic bases. Preferred bases include inorganic bases such as ammonia, alkali metal hydroxides, for example, lithium, sodium and potassium hydroxides, and alkaline earth hydroxides, for example, calcium and magnesium hydroxides, and organic bases such as mono-, di- or trialkylamines, for example, ethyl, diethyl, or triethylamine, and mono-, di- and trihydroxyalkyl amines such as, for example, 2-hydroxyethylamine, di-2-hydroxyethylamine, and tri-2-hydroxyethylamine.

Effective quantities of the compounds of the invention may be administered orally, for example, with an inert diluent or with an edible carrier. They may be enclosed in gelatin capsules or compressed into tablets. For the purpose of oral therapeutic administration, the aforesaid compounds may be incorporated with excipient and used in the form of tablets, troches, capsules, elixirs, suspensions, syrups, wafers chewing gums and the like. These preparations should contain at least 0,5% of active compound, but may be varied depending upon the particular form and may conveniently be between 4% to about 70% of the weight of the unit. The amount of active compound in such composition is such that a suitable dosage will be obtained. Preferred compositions and preparations according to the present invention are prepared so that an oral dosage unit form contains between 1.0—300 milligrams of the active compound.

The tablets, pills, capsules, troches and the like may also contain the following ingredients: a binder such as microcrystalline cellulose, gum tragancanth or geltin; an excipient such as starch or lactose, a disintegrating agent such as alginic acid, corn starch and the like; a lubricant such as magnesium stearate; a glidant such as colloidal silicon dioxide; and a sweetening agent such as sucrose or saccharin or a flavoring agent such as peppermint, methyl salicylate, or orange flavoring may be added. When the dosage unit form is a capsule, it may contain, in addition to materials of the above type, a liquid carrier such as a fatty oil. Other dosage unit forms may contain other various materials which modify the physical forms of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes and colorings and flavors. Materials used in preparing these various compositions should be pharmaceutically pure and non-toxic in the amounts used.

For the purpose of parenteral therapeutic administration, the active compound of the invention may be incorporated into a solution or suspension. These preparations should contain at least 0.1% of active compound, but may be varied between 0.5 and about 50% of the weight thereof. The amount of active compound in such compositions is such that a suitable dosage will be obtained. Preferred compositions and preparations according to the present invention are prepared so that a parenteral dosage unit contains between 0.5 to 100 milligrams of active compound.

The solutions of suspensions may also include the following components: a sterile diluent such as water for injection, saline solution, fied oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampules, disposable syrines or multiple dose vials made of glass or plastic.

While the (6,11-dihydro-11-oxidibenz[b,e]oxepinyl)pentanoic acids of the present invention exhibit antiarthritic activity in the adjuvant induced polyarthritis syndrome assay as hereinbefore discussed, the present compounds are essentially inactive antiinflammatories in an art-recognized assay for antiinflammatory activity, the carrageenen paw assay (D. E. Aultz, et al., J. Med. Chem., *20*, 66 (1977)).

The (6,11-dihydro-11-oxodibenz[b,e]oxepinyl)pentanoic acids of the present invention are also essentially inactive analgetics in an art-recognized assay for analgesia, the phenyl-para-benzoquinone assay for analgetic activity (E. Siegmund, et al., Proc. Soc., Exptl. Biol., Med. *95*, 729 (1957).

REACTION SCHEME A

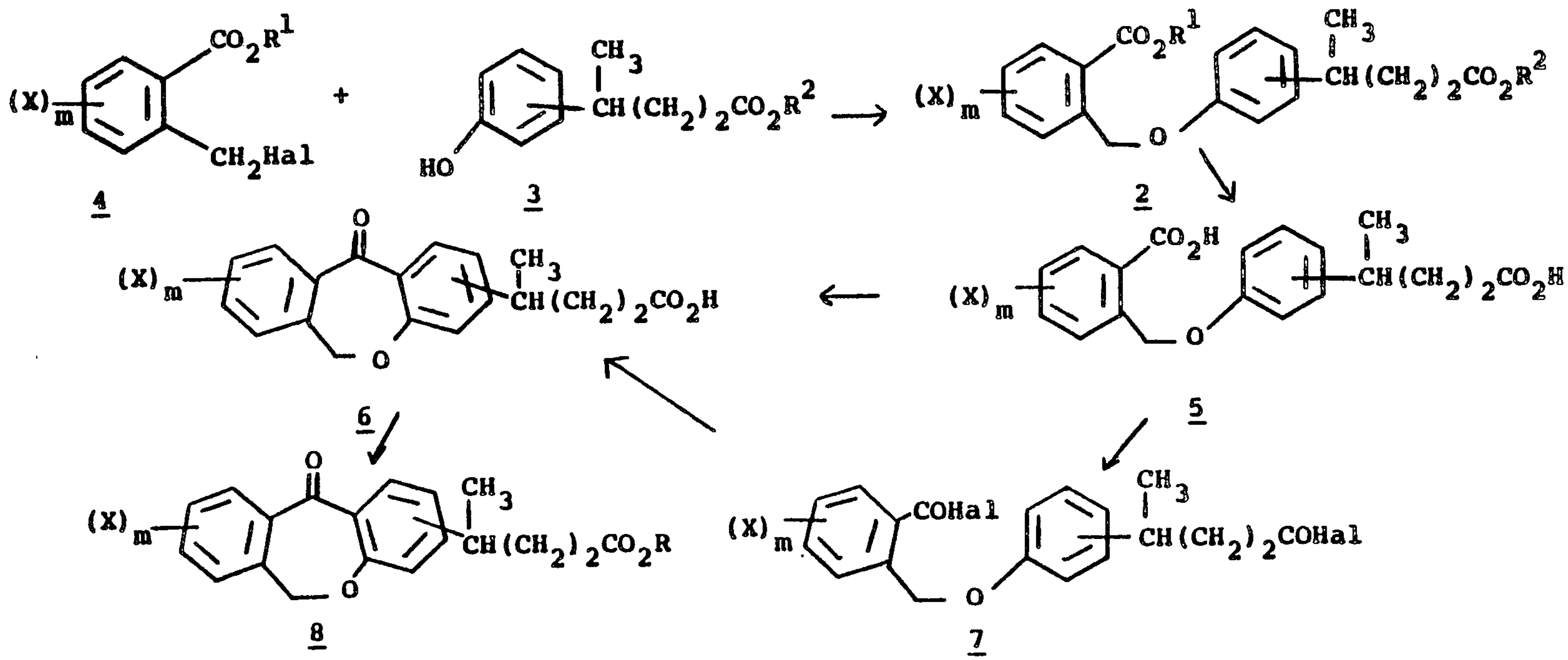

4 3 2 5 6 7 8

wherein R, $R^1$, $R^2$, X, Hal and m are as hereinbeforedescribed.

REACTION SCHEME B

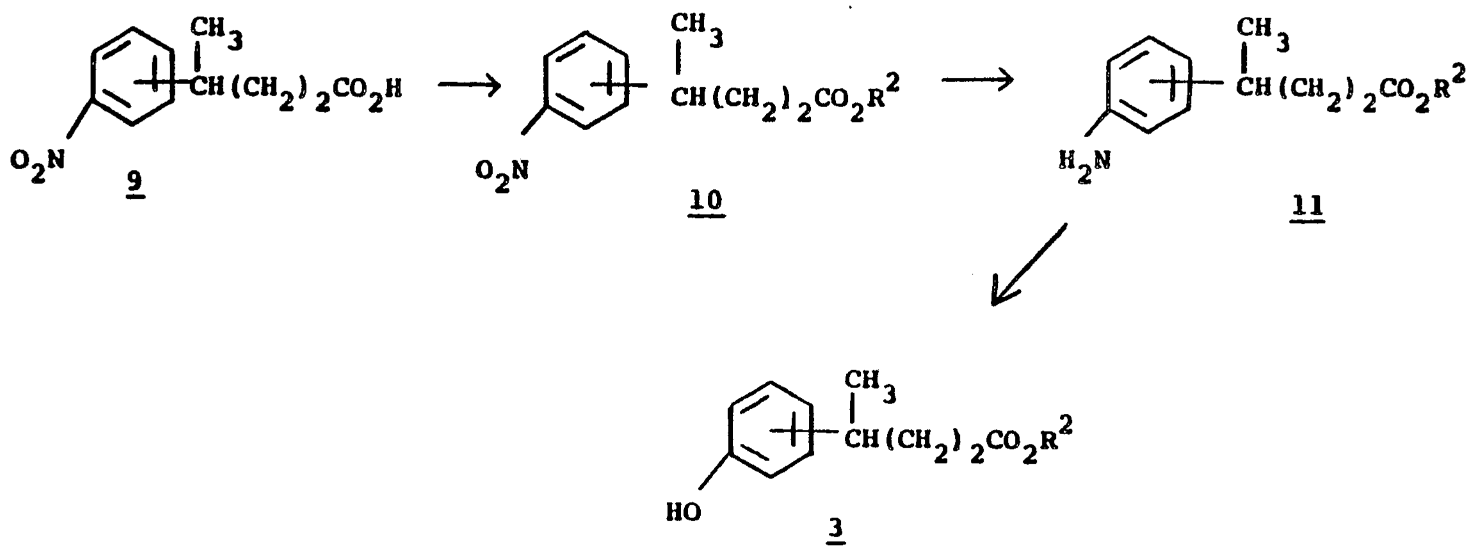

9

10

11

3

wherein $R^2$ is as hereinbeforedescribed.

The following examples are for illustration purposes only and are not to be construed as limiting the invention. All temperatures are given in degrees centigrade (°C).

Example 1

Ethyl 4-(4-nitrophenyl)pentanoate

A mixture of 110 g of 4-(4-nitrophenyl)valeric acid, 600 ml of absolute ethanol and 10 ml of concentrated sulfuric acid was heated under reflux overnight. The ethanol was evaporated *in vacuo* and the residue was partitioned between ether and water. The ether extract was washed with 5% of sodium bicarbonate solution and water, dried over anhydrous sodium sulfate, filtered and evaporated to give 119 g (97%) of product, bp. 127—128°C (1.5 mm).

Analysis:

| | | | |
|---|---|---|---|
| Calculated for $C_{13}H_{1}NO_4$: | 62.14%C | 6.82%H | 5.58%N |
| Found: | 62.28%C | 6.92%H | 5.45%N |

Example 2

Ethyl 4-(4-aminophenyl)pentanoate

A mixture of 114 g of ethyl 4-(4-nitrophenyl)pentanoate, 170 ml of absolute ethanol and 2 g of 10% of palladium-on charcoal was hydrogenated on a Paar shaker until consumption of hydrogen ceased. The catalyst was collected and the ethanol evaporated. The residue was partitioned between water and ether. The ether extract was washed with 5% of sodium bicarbonate solution and water, dried over anhydrous sodium sulfate, filtered and evaporated to give 85 g (86%) of product, bp. 118—122°C (0.55—0.60mm).

Analysis:

| | | | |
|---|---|---|---|
| Calculated for $C_{13}H_{19}NO_2$: | 70.56%C | 8.65%H | 6.33%N |
| Found: | 70.69%C | 8.64%H | 6.18%N |

Example 3

Ethyl 4-(4-hydroxyphenyyl)pentanoate

To a solution of 350 g concentrated sulfuric acid and 350 ml of water, cooled to 0°, 40 g of ethyl 4-(4-aminophenyl)pentanoate was added dropwise, maintaining the temperature at 0—2°. The mixture was treated dropwise with a solution of 13.66 g of sodium nitrite in 25 ml of water at a rate such that the temperature did not exceed 2°. The solution was added dropwise to a refluxing solution of 387 g of copper sulfate in 600 ml of water over a period of about 30 min and the resultant mixture was heated under reflux for an additional 10 min. The mixture was cooled to room temperature, 800 ml of water was added, and extracted thrice with dichloromethane (additional water was needed to dissolve the salts). The organic phase was washed with water, dried over anhydrous sodium sulfate, filtered and evaporated. The residue was distilled at 129° (0.6 mm) to give 21.05 g (53%) of product, mp 32—34°.

Analysis:

| | | |
|---|---|---|
| Calculated for $C_{13}H_{18}O_3$: | 70.25%C | 8.16%H |
| Found: | 69.98%C | 8.19%H |

Example 4

Ethyl-4-[4-(3-ethoxycarbonylbenzyloxy)phenyl]pentanoate

A mixture of 12 g of ethyl 4-(4-hydroxyphenyl)pentanoate, 16.05 g of ethyl-bromo-*o*-toluate, 0.5 g of pulverized potassium iodide and 36.49 g of potassium carbonate in 300 ml of 2-butanone was heated under reflux overnight. The salt was collected and washed with ether. The filtrate was evaporated and the residue was partitioned between water and ether. The organic layer was separated, washed with 10% sodium hydroxide solution and water, dried over anhydrous sodium sulfate, filtered and evaporated. Chromatography on a Waters Associates Prep LC-System 500 (two silica gel columns; elution with dichloromethane) gave 12.15 g (59%) of product, as an oil.

Analysis:

| | | |
|---|---|---|
| Calculated for $C_{23}H_{28}O_5$: | 71.85%C | 7.34%H |
| Found: | 71.68%C | 7.16%H |

Example 5

4-[4-(2-carboxybenzyloxy)phenyl]pentanoic acid

A mixture of 14 g of ethyl 4-[4-(2-ethoxycarbonylbenzyloxy)-phenyl]pentanoate, 31 g of potassium hydroxide in 40 ml of water and 250 ml of 95% of ethanol was heated under reflux overnight. The solution was concentrated and the residue was dissolved in water, cooled and acidified with cold conc. hydrochloric acid. The mixture was extracted with ether, and the extract was dried over anhydrous sodium sulfate, filtered and evaporated. Trituration with hexane afforded 8.9 g (72%) of product, mp. 104—106°.

Recrystallization from acetonitrile gave an analytical sample, mp 115—117.5°.

Analysis:
Calculated for $C_{19}H_{20}O_5$: 69.50%C 6.14%H
Found: 69.48%C 6.17%H

Example 6

4-(6,11-Dihydro-11-oxodibenz[b,e]oxepin-2-yl)pentanoic acid

To a solution of 6.91 g of 4-[4-(2-carboxybenzyloxy)phenyl]petanoic acid in 70 ml of dichloromethane, 13.16 g of trifluoracetic anhydride was added dropwise. The mixture was heated under reflux for five hrs. The mixture was cooled, water was added dropwise, and the resultant mixture was stirred 0.5 hrs. The organic layer was separated, washed with water, dried over anhydrous sodium sulfate, filtered and evaporated. Trituration with hexane followed by recrystallization from acetonitrile gave 3.75 g (58%) of product, mp. 120—124°.

Analysis:
Calculated for $C_{19}H_{18}O_4$: 73.53%C 5.85%H
Found: 73.69%C 5.92%H

**Claims for the Contracting States: BE CH DE FR GB IT LI LU NL SE**

1. A compound of the formula I

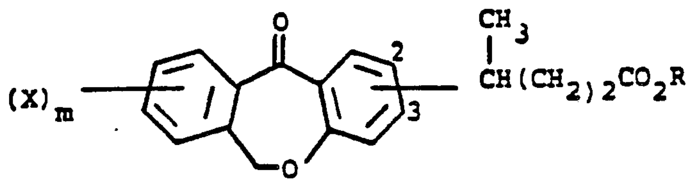

I

wherein the group

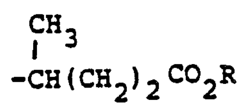

is bound to either the 2- or 3-position of the dibenz[b,e]oxepin ring, and wherein R is hydrogen or alkyl of 1 to 5 carbon atoms; X alkyl of 1 to 5 carbon atoms, alkoxy of 1 to 5 carbon atoms, halogen or trifluoromethyl; and m is 0, 1 or 2, the optical antipode thereof or a pnarmaceutically acceptable salt thereof.

2. A compound according to claim 1 wherein R is hydrogen, and m is 0.

3. The compound according to claim 2 which is 4-(6,11-dihydro-11-oxodibenz[b,e]oxepin-2-yl)pentanoic acid.

4. A pharmaceutical composition comprising as active ingredient a compound as claimed in claim 1 or a physiologically acceptable salt thereof in association with a pharmaceutically acceptable carrier.

5. Use of a compound as claimed in claim 1 or a physiologically acceptable salt thereof for the manufacture of a medicament having antiarthritic activity.

6. A compound of the formula

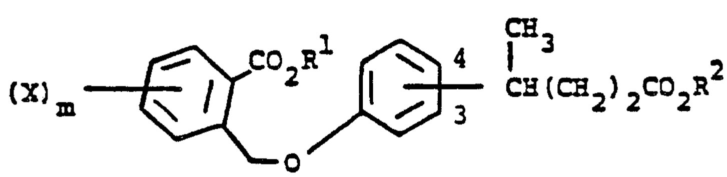

II

wherein the group

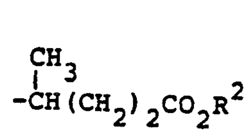

is bound to either the 3- or 4-position wherein $R^1$ is hydrogen or alkyl of 1 to 5 carbon atoms; $R^2$ is hydrogen or alkyl of 1 to 5 carbon atoms with the proviso that $R^1$ and $R^2$ are simultaneously hydrogen or alkyl of 1 to 5 carbon atoms; X is alkyl of 1 to 5 carbon atoms, halogen or trifluoromethyl; and m is 0, 1 or 2, or the optical antipode thereof.

7. A compound as claimed in claim 6 wherein $R^1$ and $R^2$ are alkyl of 1 to 5 carbon atoms, and m is 0.

8. A process for the preparation of a compound as claimed in claim 1 which comprises cyclizing a compound of the formula

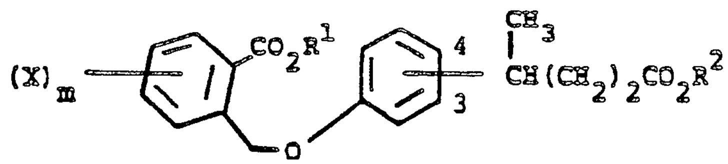

II

wherein the group

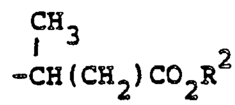

is bound either to the 3- or 4-position and wherein $R^1$ is hydrogen or alkyl of 1 to 5 carbon atoms, $R^2$ is hydrogen or alkyl of 1 to 5 carbon atoms with the proviso that $R^1$ and $R^2$ are simultaneously hydrogen or alkyl of 1 to 5 carbon atoms, X is alkyl of 1 to 5 carbon atoms, halogen or trifluoromethyl and m is 0, 1 or 2, or the optical antipode thereof, in the presence of a dehydrating agent, or cyclizing a compound of the formula

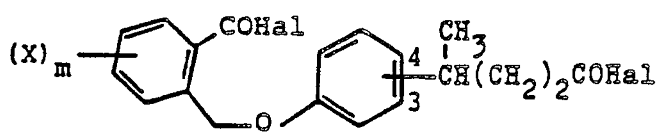

III

wherein the group

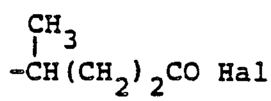

is bound either to the 2- or 4-position, and wherein X and m are as defined above, in the presence of a Friedel-Crafts reagent and hydrolizing the compound obtained, and optionally esterifying a compound of the formula I wherein $R^2$ is hydrogen.

9. A process as claimed in claim 8 wherein the cyclization is carried through in the presence of trifluoroacetic anhydride, polyphosporic acid, polyphosphoric-alkanol, phosphorus pentoxide or phosphorus pentoxide-alkanoic acid as a dehydrating agent, or in the presence of aluminum chloride, stannic chloride or ferric chloride as a Friedel-Crafts reagent.

10. A process as claimed in claim 9 wherein the cyclization is carried through at a temperature up to 120°C in the presence or absence of a solvent.

**Claims for the Contracting State: AT**

1. A process for the preparation of a compound of the formula

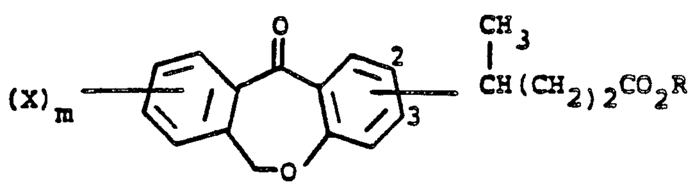

I

wherein the group

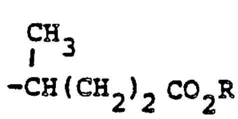

is bound to either the 2- or 3-position and wherein R is hydrogen or alkyl of 1 to 5 carbon atoms; X alkyl of 1 to 5 carbon atoms, alkoxy of 1 to 5 carbon atoms, halogen or trifluoromethyl; and m is 0, 1 or 2, the optical antipode thereof or a pharmaceutically acceptable salt thereof, which comprises cyclizing a compound of the formula

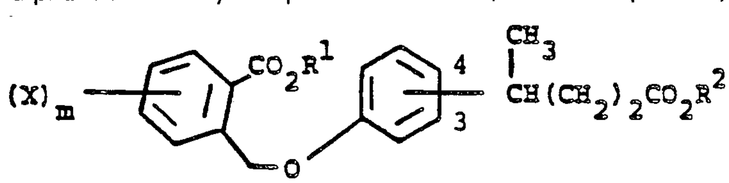

II

wherein the group

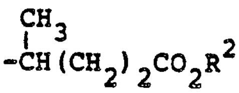

is bound to either the 3- or 4-position, and wherein $R^1$ is hydrogen or alkyl of 1 to 5 carbon atoms; $R^2$ is hydrogen or alkyl of 1 to 5 carbon atoms with the proviso that $R^1$ and $R^2$ are simultaneously hydrogen or alkyl of 1 to 5 carbon atoms; X is alkyl of 1 to 5 carbon atoms, halogen or trifluoromethyl; and m is 0, 1 or 2, or the optical antipode thereof, in the presence of a dehydrating agent, or cyclizing a compound of the formula

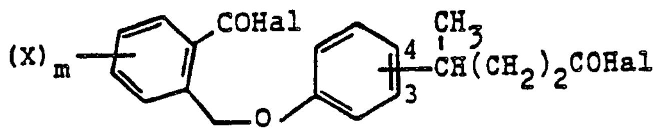 III

wherein the group

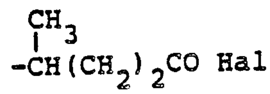

is bound either to the 3- or 4-position and wherein X and m are as defined above in the presence of a Friedel-Crafts reagent and hydrolizing the compound obtained, and optionally esterifying a compound of the formula I wherein $R^2$ is hydrogen.

2. A process as claimed in claim 1 wherein R is hydrogen and m is 0.

3. A process as claimed in claim 2 wherein the compound 4-(6,11-dihydro-11-oxodibenz[b,e]oxepin-2-yl)pentanoic acid is prepared.

4. A process for the preparation of a compound of the formula

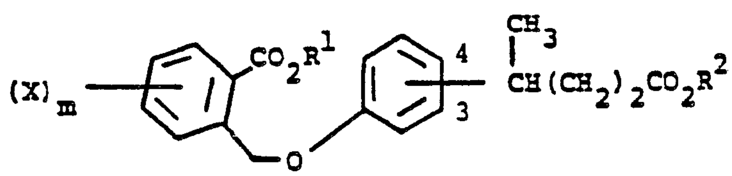 II

wherein the group

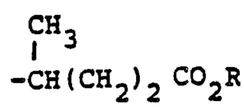

is bound either to the 3- or 4-position, and wherein $R^1$ and $R^2$ are alkyl of 1 to 5 carbon atoms; X is alkyl of 1 to 5 carbon atoms, alkoxy of 1 to 5 carbon atoms, halogen or trifluoromethyl; and m is 0, 1 or 2, or an optical antipode thereof, which comprises reacting a compound of the formula

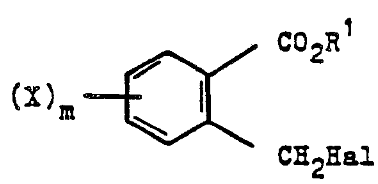

wherein $R^1$ is akyl of 1 to 5 carbon atoms, Hal is halogen, X is alkyl of 1 to 5 carbon atoms, alkoxy of 1 to 5 carbon atoms, halogen or trifluoromethyl; and m is 0, 1 or 2, with a compound of the formula

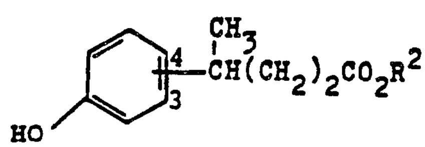

wherein the group

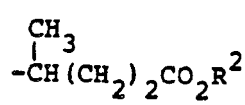

is bound either to the 3- or 4-position, and wherein $R^2$ is alkyl of 1 to 5 carbon atoms, or an optical antipode thereof, in an alkanone in the presence of an alkali metal carbonate.

**Patentansprüche für die Vertragsstaaten: BE CH DE FR GB IT LI LU NL SE**

1. Eine Verbindung der Formel I

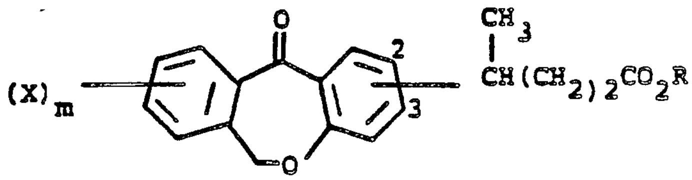

I

wobei die Gruppe

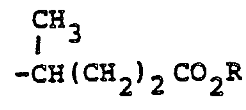

entweder an die 2- oder an die 3-Stellung des Dibenz[b,e]oxepinringes gebunden ist, und worin R Wasserstoff oder Akyl mit 1 bis 5 Kohlenstoffatomen ist; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; der optische Antipode davon; oder ein pharmazeutisch annehmbares Salz davon.

2. Eine Verbindung nach Anspruch 1, wobei R Wasserstoff ist, und m 0 ist.

3. Die Verbindung nach Anspruch 2, welche 4-(6,11-Dihydro-11-oxodibenz[b,e]oxepin-2-yl)pentansäure ist.

4. Eine pharmazeutische Zusammensetzung, enthaltend als Wirkbestandteil eine wie in Anspruch 1 beanspruchte Verbindung, oder ein physiologisch annehmbares Salz davon, gemeinsam mit einem pharmazeutisch annehmbaren Träger.

5. Verwendung einer wie in Anspruch 1 beanspruchten Verbindung oder eines physiologisch annehmbaren Salzes davon, für die Herstellung eines Arzneimittels mit antiarthritischer Wirksamkeit.

6. Eine Verbindung der Formel

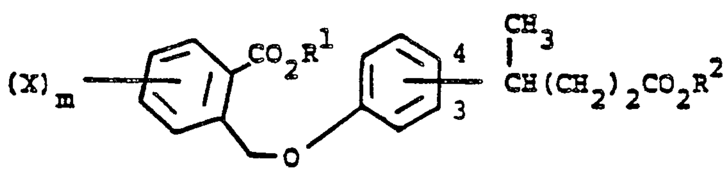

II

wobei die Gruppe

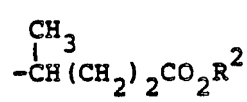

entweder an die 3- oder an die 4-Stellung gebunden ist, und worin $R^1$ Wasserstoff oder Alkyl mit 1 bis 5 Kohlenstoffatomen ist; $R^2$ Wasserstoff oder Alkyl mit 1 bis 5 Kohlenstoffatomen ist, mit er Maßgabe, daß $R^1$ und $R^2$ gleichzeitig Wasserstoff oder Alkyl mit 1 bis 5 Kohlenstoffatomen sind; X Alkyl mit 1 bis 5 Kohlenstoffatomen, Halogen oder Trifluormethyl ist; und m 0, 1 oder 2 ist; oder der optische Antipode davon.

7. Eine Verbindung wie in Anspruch 6 beansprucht, wobtei $R^1$ und $R^2$ Alkyl mit 1 bis 5 Kohlenstoffatomen sind, und m 0 ist.

8. Ein Verfahren zur Herstellung einer wie in Anspruch 1 beanspruchten Verbindung, welches das Cyclisieren einer Verbindung der Formel

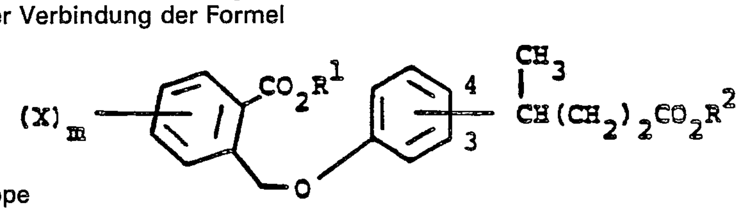

II

wobei die Gruppe

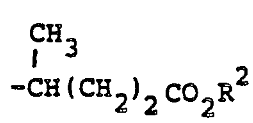

entweder an die 3- oder an die 4-Stellung gebunden ist, und worin $R^1$ Wasserstoff oder Alkyl mit 1 bis 5 Kohlenstoffatomen ist; $R^2$ Wasserstoff oder Alkyl mit 1 bis 5 Kohlenstoffatomen ist, mit der Maßgabe, daß $R^1$ und $R^2$ gleichzeitig Wasserstoff oder Alkyl mit 1 bis 5 Kohlenstoffatomen sind; X Alkyl mit 1 bis 5 Kohlenstoffatomen, Halogen oder Trifluormethyl ist; und m 0, 1 oder 2 ist; oder des optischen Antipoden davon, in Gegenwart eines Dehydratisierungsmittels, oder das Cyclisieren einer Verbindung der Formel

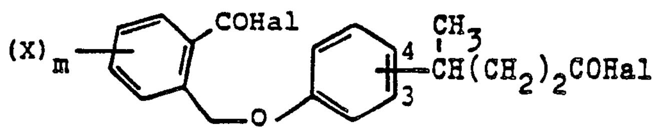
III

wobei die Gruppe

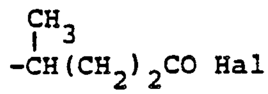

entweder an die 3- oder an die 4-Stellung gebunden ist, und worin X und m wie oben definiert sind, in Gegenwart eines Friedel-Crafts-Reagens, und das Hydrolysieren der erhaltenen Verbindung, und gegebenenfalls das Verestern einer Verbindung der Formel I, worin $R^2$ Wasserstoff ist, umfaßt.

9. Ein Verfahren wie in Anspruch 8 beansprucht, wobei die Cyclisierung in Gegenwart von Trifluoressigsäureanhydrid, Polyphosphorsäure, Polyphosphorsäure-Alkanol, Phosphorpentoxid oder Phosphorpentoxid-Alkansäure als einem Dehydratisierungsmittel, oder in der Gegenwart von Aluminiumchlorid, Stannichlorid oder Ferrichlorid als ein Friedel-Crafts-Reagens, durchgeführt wird.

10. Ein Verfahren wie in Anspruch 9 beansprucht, wobei die Cyclisierung bei einer Temperatur bis zu 120°C in Gegenwart oder Abwesenheit eines Lösungsmittels durchgeführt wird.

**Patentansprüche für den Vertragsstaat: AT**

1. Ein Verfahren zur Herstellung einer Verbindung der Formel

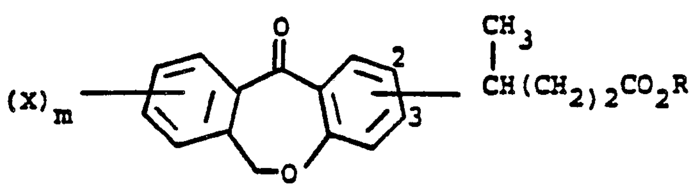
I

wobei die Gruppe

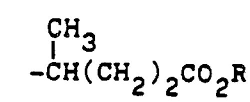

entweder an die 2- oder an die 3-Stellung gebunden ist, und worin R Wasserstoff oder Akyl mit 1 bis 5 Kohlenstoffatomen ist; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; der optischen Antipoden davon; oder eines pharmazeutisch annehmbaren Salzes davon, welches das Cyclisieren einer Verbindung der Formel

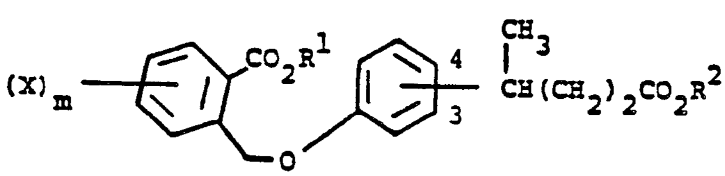
II

wobei die Gruppe

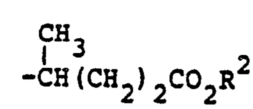

entweder an die 3- oder an die 4-Stellung gebunden ist, und worin $R^1$ Wasserstoff oder Alkyl mit 1 bis 5 Kohlenstoffatomen ist; $R^2$ Wasserstoff oder Alkyl mit 1 bis 5 Kohlenstoffatomen ist, mit der Maßgabe, daß $R^1$ und $R^2$ gleichzeitig Wasserstoff oder Alkyl mit 1 bis 5 Kohlenstoffatomen sind; X Alkyl mit 1 bis 5 Kohlenstoffatomen, Halogen oder Trifluormethyl ist; und m 0, 1 oder 2 ist; oder des optischen Antipoden davon, in Gegenwart eines Dehydratisierungsmittels, oder das Cyclisieren einer Verbindung der Formel

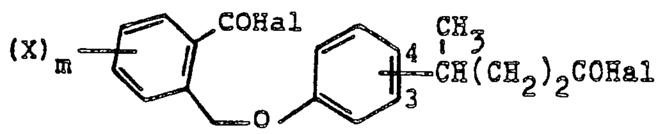 III

wobei die Gruppe

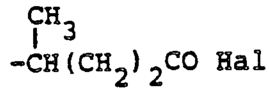

entweder an die 3- oder an die 4-Stellung ist, und worin X uund m wie oben definiert sind, in Gegenwart eines Friedel-Crafts-Reagens, und das Hydrolysieren der erhaltenen Verbindung, und gegebenenfalls das Verestern einer Verbindung der Formel I, worin $R^2$ Wasserstoff ist, umfaßt.

2. Ein Verfahren wie in Anspruch 1 beansprucht, wobei R Wasserstoff ist, und m 0 ist.

3. Ein Verfahren wie in Anspruch 2 beansprucht, wobei die Verbindung 4-(6,11-Dihydro-11-oxodibenz[b,e]oxepin-2-yl)pentansäure hergestellt wird.

4. Ein Verfahren zur Herstellung einer Verbindung der Formel

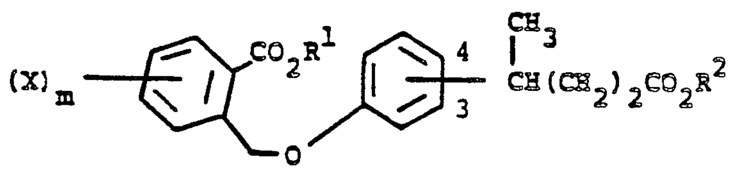 II

wobei die Gruppe

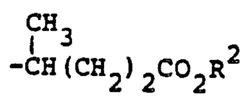

entweder an die 3- oder an die 4-Stellung gebunden ist, und worin $R^1$ und $R^2$ Alkyl mit 1 bis 5 Kohlenstoffatomen sind; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; oder eines optischen Antipoden davon; welches das Umsetzen einer Verbindung der Formel

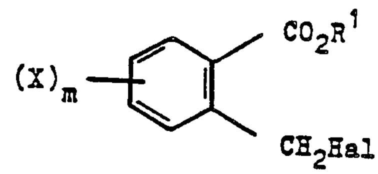

worin $R^1$ Alkyl mit 1 bis 5 Kohlenstoffatomen ist; Hal Halogen ist; X Alkyl mit 1 bis 5 Kohlenstoffatomen, Alkoxy mit 1 bis 5 Kohlenstoffatomen, Halogen oder Trifluormethyl ist; und m 0, 1 oder 2 ist; mit einer Verbindung der Formel

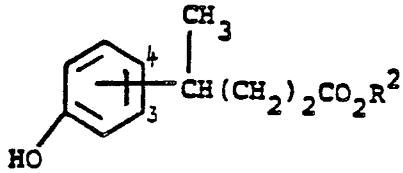

wobei die Gruppe

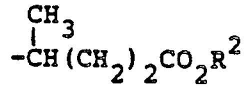

entweder an die 3- oder an die 4-Stellung gebunden ist, und worin $R^2$ Alkyl mit 1 bis 5 Kohlenstoffatomen ist, oder eines optischen Antipoden davon, in einem Alkanon in Gegenwart eines Alkalimetallcarbonats umfaßt.

**Revendications pour les Etats contractants: BE CH DE FR GB IT LI LU NL SE**

1. Composé de formule I:

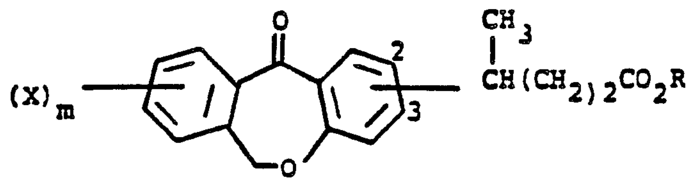

I

dans laquelle le groupe

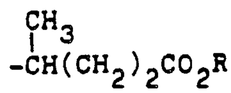

est lié au niveau de la position 2- ou 3- du cycle dibenzo [b,e]oxépine, et dans laquelle R représente un atome d'hydrogène ou un groupe alkyle comportant de 1 à 5 atomes de carbone; X représente un groupe alkyle comportant de 1 à 5 atomes de carbone, un groupe alkoxy comportant de 1 à 5 atomes de carbone, un atome d'halogène ou un groupe trifluorométhyle; et m est égal à 0, 1 ou 2; son énantiomère; ou un sel pharmaceutiquement acceptable de celui ci.

2. Composé selon la revendication 1, dans laquel R représente un atome d'hydrogène, et m est égal à 0.

3. Composé selon la revendication 2, qui est le 4-(6,11-dihydro-11-oxodibenzo[b,e]oxépin-2-yl)pentanoïque.

4. Composition pharmaceutique, comprenant, comme ingrédient actif, un composé selon la revendication 1, ou un sel physiologiquement acceptable de celui-ci associé à un véhicule pharmaceutiquement acceptable.

5. Utilisation d'un composé selon la revendication 1 ou d'un sel physiologiquement acceptable de celui-ci pour la fabrication d'un médicament ayant une activité anti-arthritique.

6. Composé de formule:

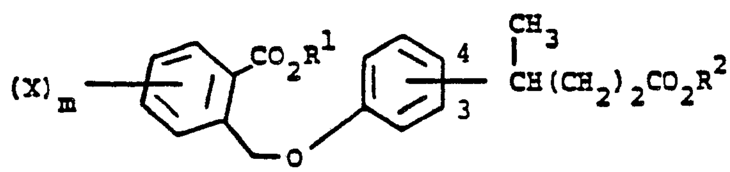

II

dans laquelle le groupe

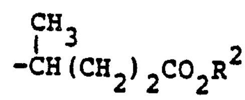

est lié au niveau de la position 3- ou 4, et dans laquelle $R^1$ représente un atome d'hydrogène ou un groupe alkyle comportant de 1 à 5 atomes de carbone; $R^2$ représente un atome d'hydrogène ou un groupe alkyle comportant de 1 à 5 atomes de carbone, à condition que les groupes $R^1$ et $R^2$ représentent simultanément des atomes d'hydrogène ou des groupes alkyle comportant de 1 à 5 atomes de carbone; X représente un groupe alkyle comportant de 1 à 5 atomes de carbone, un atome d'halogène ou un groupe trifluorométhyle, et m est égal à 0, 1 ou 2; ou son énantiomère.

7. Composé selon la revendication 6, dans lequel $R^1$ et $R^2$ sont des groupes alkyle comportant de 1 à 5 atomes de carbone et m est égal à 0.

8. Procédé de préparation d'un composé selon la revendication 1, dans lequel on cyclise un composé de formule:

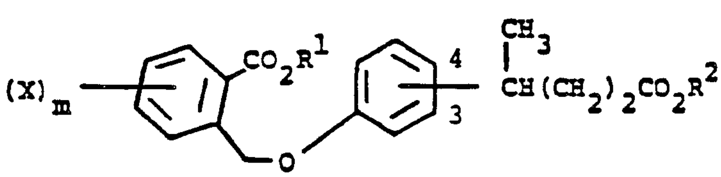

II

dans laquelle le groupe

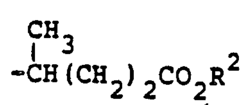

est lié au niveau de la position 3- ou 4-, et dans laquelle $R^1$ représente un atome d'hydrogène ou un groupe

alkyle comportant de 1 à 5 atomes de carbone, $R^2$ représente un atome d'hydrogène ou un grupe alkyle comportant de 1 à 5 atomes de carbone, à condition que $R^1$ et $R^2$ représentent simultanément des atomes d'hydrogène ou des groupes alkyle comportant de 1 à 5 atomes de carbone, X représente un groupe alkyle comportant de 1 à 5 atomes de carbone, un atome d'halogène ou un groupe trifluorométhyle, et m est égal à 0, 1 ou 2, ou son énantiomère, en présence d'un agent déshydratant; ou on cyclise un composé de formule:

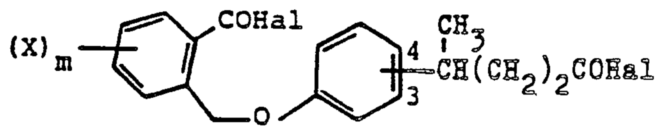 III

dans laquelle le groupe

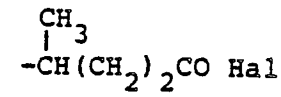

est lié au niveau de la position 3- ou 4-, et dans laquelle X et m sont tels que définis ci-desus, en présence d'un réactif de Friedel et Crafts, et on hydrolyse le composé obtenu, et on estérifie éventuellement un composé de formule I dans laquelle $R^2$ représente un atome d'hydrogène.

9. Procédé selon la revendication 8, dans lequel la cyclisation est effectuée en présence d'anhydride trifluoroacétique, d'acide polyphosphorique, d'acide polyphosphorique associé à un alcanol, de pentoxyde de phosphore ou de pentoxyde de phosphore associé à un acide alcanoïque en tant qu'agent déshydratant, ou en présence de chlorure d'aluminium, de chlorure stannique ou de chlorure ferrique en tant que réactif de Friedel et Crafts.

10. Procédé selon la revendication 9, dans lequel la cyclisation est effectuée à une température allant jusqu'à 120°C en présence ou en l'absence d'un solvant.

**Revendications pour l'Etat contractant: AT**

1. Procédé de préparation d'un composé de formule:

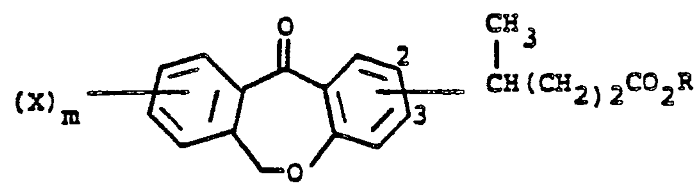 I

dans laquelle le groupe

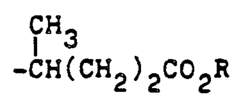

est lié au niveau de la position 2- ou 3-, et dans laquelle R représente un atome d'hydrogène ou un groupe alkyle comportant de 1 à 5 atomes de carbone; X représente un groupe alkyle comportant de 1 à 5 atomes de carbone, un groupe alkoxy comportant de 1 à 5 atomes de carbone, un atome d'halogène ou un groupe trifluorométhyle; et m est égal à 0, 1 ou 2; son énantiomère ou un sel pharmaceutiquement acceptable de celui-ci; dans lequel on cyclise un composé de formule:

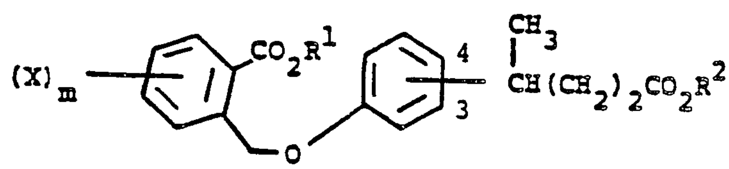 II

dans laquelle le groupe

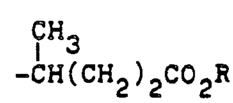

est lié au niveau de la position 3- ou 4-, et dans laquelle $R^1$ représente un atome d'hydrogène ou un groupe alkyle comportant de 1 à 5 atomes de carbone, $R^2$ représente un atome d'hydrogène ou un groupe alkyle comportant de 1 à 5 atomes de carbone, à condition que $R^1$ et $R^2$ représentent simultanément des atomes d'hydrogène ou des groupes alkyle comportant de 1 à 5 atomes de carbone, X représente un groupe alkyle comportant de 1 à 5 atomes de carbone, un atome d'halogène ou un groupe trifluorométhyle, et m est égal

à 0, 1 ou 2, ou son énantiomère, en présence d'un agent déshydratant; ou on cyclise un composé de formule

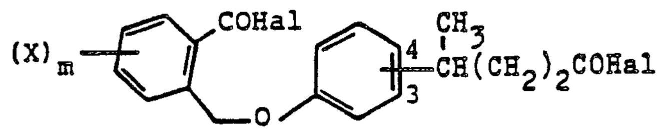 III

dans laquelle le groupe

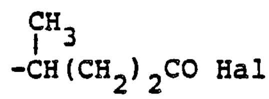

est lié au niveau de la position 3- ou 4, et dans laquelle X et m sont tels que définis ci-dessus, en présence d'un reactif de Friedel et Crafts, et on hydrolyse le composé obtenue, et on estérifie éventuellement un composé de formule I dans laquelle $R^2$ représente un atome d'hydrogène.

2. Procédé selon la revendication 1, dans lequel R représente un atome d'hydrogène et m est égal à 0.

3. Procédé selon la revendication 2, dans lequel le composé qui est préparé, est l'acide 4-(6,11-dihydro-11-oxodibenzo[b,e]oxépin-2-yl)pentanoïque.

4. Procédé de préparation d'un composé de formule:

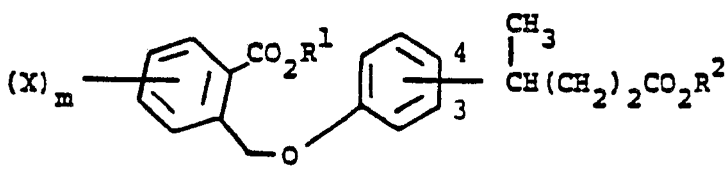 II

dans laquelle le groupe

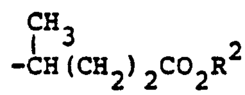

est lié au niveau de la position 3- ou 4-, et dans laquelle les groupes $R^1$ et $R^2$, représentent des groupes alkyle comportant de 1 à 5 atomes de carbone; X représente un groupe alkyle comportant de 1 à 5 atomes de carbone, un groupe alkoxy comportant de 1 à 5 atomes de carbone, un atome d'halogène ou un groupe trifluorométhyle; et m est égal à 0, 1 ou 2; ou son énantiomère; dans lequel on fait réagir un composé de formule:

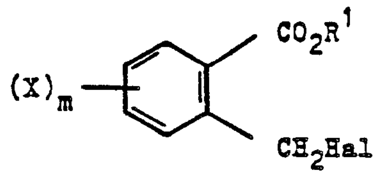

dans laquelle $R^1$ représente un groupe alkyle comportant de 1 à 5 atomes de carbone, Hal représente un atome d'halogène, X représente un groupe alkyle comortant de 1 à 5 atomes de carbone, un groupe alkoxy comportant de 1 à 5 atomes de carbone, un atome d'halogène ou un groupe trifluorométhyle; et m est égal à 0, 1 ou 2, avec un composé de formule:

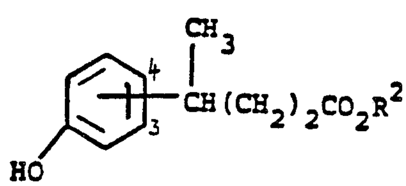

dans laquelle le groupe

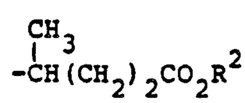

est lié au niveau de la position 3- ou 4-, et dans laquelle $R^2$ représente un groupe alkyle comportant de 1 à 5 atomes de carbone, ou son enantiomère, dans un alcanone en présence d'un carbonate de métal alcalin.